(19) Europäisches Patentamt
European Patent Office
Office européen des brevets

(11) Numéro de publication : 0 232 348 B1

(12) # FASCICULE DE BREVET EUROPEEN

(45) Date de publication du fascicule du brevet : 12.06.91 Bulletin 91/24

(51) Int. Cl.$^5$ : G01N 33/542

(21) Numéro de dépôt : 86904837.1

(22) Date de dépôt : 31.07.86

(86) Numéro de dépôt international : PCT/FR86/00269

(87) Numéro de publication internationale : WO 87/00927 12.02.87 Gazette 87/04

(54) **PROCEDE HOMOGENE DE DETECTION ET/OU DE DETERMINATION PAR LUMINESCENCE D'UN ANALYTE DANS UN MILIEU SUSCEPTIBLE DE LE CONTENIR.**

(30) Priorité : 02.08.85 FR 8511905

(43) Date de publication de la demande : 19.08.87 Bulletin 87/34

(45) Mention de la délivrance du brevet : 12.06.91 Bulletin 91/24

(84) Etats contractants désignés : AT BE CH DE GB IT LI LU NL SE

(56) Documents cités :
EP-A- 0 103 558
US-A- 4 261 968

(73) Titulaire : CIS BIO INTERNATIONAL
RN 306
F-91000 Saclay (FR)

(72) Inventeur : MATHIS, Gérard
17, impasse de la Capelle des Ladres
F-30200 Bagnols-s/Ceze (FR)
Inventeur : DAVIN, Thierry
37, résidence les Lavandins
F-84840 Lapalud (FR)

(74) Mandataire : Gillard, Marie-Louise et al
Cabinet Beau de Loménie 55, Rue d'Amsterdam
F-75008 Paris (FR)

EP 0 232 348 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne un procédé homogène de détection et/ou de détermination d'un analyte dans un milieu susceptible de le contenir.

La détermination de la présence ou de la concentration de substances organiques ou biologiques circulantes dans des liquides biologiques est une étape importante dans le diagnostic de nombreuses maladies.

Une des méthodes couramment utilisées pour cette détermination est basée sur la formation d'un complexe entre l'analyte, c'est-à-dire la substance à détecter ou à doser, et un récepteur de celui-ci, qui est une substance capable de se fixer spécifiquement à l'analyte. Le complexe ainsi formé est mis en évidence par un réactif marqué.

Cette méthode englobe les procédés de dosages immunologiques dits par "compétition" ou "par excès" décrits par exemple par R. EKINS dans "Monoclonal Antibodies and Development in Immunoassay" Elsevier 1981 p. 3-21. Le marquage du réactif mis en oeuvre est réalisé notamment à l'aide d'un élément radioactif, d'une enzyme ou d'un composé luminescent par exemple fluorescent, chimioluminescent ou phosphorescent. On parle alors de procédés radioimmunologiques, immunoenzymatiques ou immunologiques par luminescence (fluorescence, phosphorescence ou chimioluminescence).

Dans les procédés de dosages immunologiques dits par compétition on fait incuber le milieu susceptible de contenir l'analyte recherché avec un récepteur de celui-ci en défaut en présence d'une quantité donnée de l'analyte marqué.

Il se produit alors une compétition entre l'analyte recherché et l'analyte marqué vis-à-vis du récepteur. On procède ensuite à la séparation de la fraction contenant l'analyte marqué lié de la fraction contenant l'analyte marqué libre et on mesure la quantité d'analyte marqué dans l'une ou l'autre des fractions.

Dans les procédés de dosage dits par excès on met en oeuvre deux récepteurs de spécificité différente pour l'analyte recherché, l'un d'entre eux étant marqué. Ils nécessitent aussi une étape de séparation de la fraction contenant le récepteur marqué lié de celle contenant le récepteur marqué libre.

Afin de réaliser rapidement les dosages avec une très grande sensibilité, on a cherché différents moyens pour supprimer l'étape de séparation et mis au point des procédés dits "homogènes".

Dans le domaine des dosages immunologiques par fluorescence, il existe relativement peu de procédés homogènes.

Toute méthode homogène de fluorescence est basée sur le fait que la liaison du récepteur marqué avec l'analyte induit une modification des caractéristiques d'émission de la molécule fluorescente.

Par exemple en polarisation de fluorescence, on mesure la polarisation de la lumière émise, laquelle varie avec la taille de l'édifice moléculaire portant la molécule fluorescente.

Un autre procédé homogène basé sur le phénomène du transfert d'énergie entre deux chromophores est décrit dans le brevet FR 2 282 115. Dans ce procédé, le transfert d'énergie du chromophore donneur vers le chromophore accepteur se produit si le spectre d'émission du premier et celui d'excitation du second se recouvrent (compatibilité en énergie) et si la distance entre les deux chromophores est inférieure en général à 100 Å. De même, le procédé décrit dans le brevet FR 2 422 165 met en oeuvre un marqueur chimioluminescent et un agent d'extinction capable de modifier l'émission de la lumière par chimioluminescence lorsque cette molécule se trouve à une distance courte mais ne provoquant pas une collision, en général inférieure à 100 Å.

Ces procédés présentent toutefois des inconvénients. Dans le cas de la polarisation, il existe des limitations liées à la taille de l'analyte recherché. Dans le procédé utilisant le transfert d'énergie, il faut marquer à la fois l'analyte et le récepteur et l'émission de luminescence de l'accepteur interfère sur la mesure de la luminescence du donneur. D'autre part, on ne peut pas choisir dans ce cas n'importe quel couple de chromophores (compatibilité en énergie).

On peut également citer les procédés homogènes de dosages par fluorescence décrits dans le brevet US 4 318 707 et la demande de brevet EP 17908 au nom de SYVA.

Le procédé décrit dans le brevet US 4 318 707 met en oeuvre des particules qui sont capables de réduire l'intensité d'excitation et/ou de réduire l'intensité d'émission de molécules excitables électroniquement.

Le procédé selon la demande de brevet EP 17908 est basé sur la capacité de répartition d'une substance chromogène entre une fraction où le chromogène retient son activité chromogène et une fraction où l'activité chromogène est inhibée, le degré de répartition étant fonction de la concentration de l'analyte dans le milieu de dosage.

Par ailleurs, il est connu que la présence d'un atome lourd, tel qu'un atome d'iode, à proximité ou à l'intérieur de la structure d'un composé fluorescent induit une diminution de sa fluorescence.

Cet effet très général est décrit dans la littérature par exemple par E.L. WEHRY dans "Fluorescence" éd. G.G. Guilbault M. Dekker N.Y. 1967. Selon l'état de la technique, cet effet est observé lorsque la molécule fluorescente contient naturellement un atome lourd, lorsque des atomes lourds sont introduits chimiquement dans

la molécule fluorescente ou lorsque des atomes lourds sont présents en solution dans le milieu de mesure. Le mécanisme de cet effet d'atome lourd n'est pas très bien connu dans le cas où il est extérieur à la molécule. Toutefois, dans le cas où la molécule fluorescente contient des atomes lourds (présents naturellement ou incorporés par voie chimique), on explique ce phénomène par une augmentation des couplages spin-orbite de ladite molécule fluorescente par rapport à son homologue ne contenant pas d'atome lourd, qui peut se traduire par un accroissement des transitions inter-sytèmes non radiatives $S_1^* \rightsquigarrow T_1^*$, radiatives $T_1^* \rightarrow S_0$, mais aussi par des conversions internes non radiatives $S_1^* \rightsquigarrow S_0$. On observe dans tous les cas une diminution de la fluorescence due à la présence d'un atome lourd et, quand les molécules et les conditions de mesure le permettent, une modification de la phosphorescence. Un exemple concret de cet effet est la diminution du rendement quantique de la fluorescéine observée lorsque celle-ci est liée chimiquement à des molécules polyiodées, telles que la triiodothyronine ($T_3$) ou la tétraiodothyronine ($T_4$) ; il s'agit d'un effet d'atome lourd interne.

Dans le cas où l'atome lourd n'est pas fixé par liaison chimique à la molécule fluorescente mais présent en solution dans le milieu de mesure, on pense que l'augmentation du couplage spin-orbite dans la molécule luminescente serait due soit aux collisions de la molécule fluorescente avec l'atome lourd, soit à la formation de faibles complexes par transfert de charge. Elle se manifeste dans les faits par une augmentation des transitions intersystèmes de la molécule luminescente $S_1^* \rightsquigarrow T_1^*$ et $T_1^* \rightarrow S_0$, de sorte que si l'effet d'atome lourd se traduit tantôt par une augmentation, tantôt par une diminution de l'intensité des molécules phosphorescentes il se traduit toujours par une diminution de l'intensité des molécules fluorescentes.

Cet effet d'atome lourd interne a déjà été utilisé dans un procédé homogène de dosage par fluorimétrie. A cet effet, on peut faire référence à la demande de brevet EP 15695 qui décrit un tel procédé dans lequel on met en oeuvre un conjugué formé entre un analogue de ligand et un composé fluorescent, l'analogue de ligand portant naturellement un atome lourd capable d'éteindre ladite molécule fluroescente, ledit conjugué étant en liaison de covalence avec un polysaccharide macromoléculaire. Dans ce cas, la liaison de l'anticorps spécifique avec la molécule contenant l'atome lourd réduit l'inhibition et se traduit par une augmentation de la fluorescence.

On a maintenant trouvé que l'effet d'atome lourd pouvait être mis à profit dans un procédé homogène de détection et/ou de détermination par luminescence d'un analyte sans que l'atome lourd soit en solution dans le milieu de mesure ou fixé sur la molécule luminescente.

On a en effet constaté, de façon surprenante, qu'il se produit, dans un dosage immunologique par compétition ou par excès, une transition intersystème sur la molécule luminescente lorsque la molécule luminescente est liée à l'un des réactifs mis en oeuvre alors que l'autre réactif porte des motifs contenant au moins un atome lourd.

Sous son aspect général, la présente invention concerne donc un procédé de détection et/ou de détermination d'un analyte dans un milieu susceptible de le contenir, par mise en évidence du produit de la réaction entre l'analyte et au moins un récepteur correspondant qui consiste :

1) à ajouter audit milieu un premier réactif constitué d'un récepteur dudit analyte ;

2) à ajouter au second réactif constitué d'au moins un des éléments du produit de la réaction de l'analyte avec au moins l'un de ses récepteurs, l'un des deux réactifs étant couplé avec un composé luminescent et l'autre réactif étant couplé avec un atome lourd ou des motifs contenant un atome lourd ;

3) à faire incuber le milieu résultant soit après l'addition de chaque réactif soit après l'addition desdits réactifs ;

4) à exciter le milieu résultant, et

5) à mesurer, à l'équilibre ou en cinétique, le signal émis par le composé luminescent, ledit signal étant modulé par effet d'atome lourd.

Dans la présente description on définit par :

– "analyte" toute substance ou groupe de substances analogues à détecter et/ou déterminer ;

– "récepteur" toute substance capable de se fixer spécifiquement sur un site dudit analyte ;

– "composé luminescent'" toute substance, qui excitée à une longueur d'onde donnée ou par un composé chimique donné, est capable d'émettre de la lumière ;

– "atome lourd" un atome de nombre atomique élevé et dont la présence à proximité d'une molécule luminescente est capable d'induire une augmentation du couplage spin-orbite de celle-ci. A titre d'exemples d'atomes lourds appropriés aux fins de l'invention, on peut citer notamment les atomes d'halogènes, le mercure, le thallium, le plomb, l'argent ;

– "motif contenant au moins un atome lourd" toute substance chimique comportant naturellement au moins un atome lourd ou sur laquelle on peut fixer au moins un atome lourd.

D'autre part, l'expression "élément du produit de la réaction de l'analyte avec au moins l'un de ses récepteurs" désigne l'analyte ou au moins l'un de ses récepteurs selon le type de méthodes utilisées.

L'analyte peut être de nature biologique ou non. Il englobe notamment les substances biologiques telles que les anticorps, les antigènes, les toxines, les enzymes, les protéines, par exemple la protéine A, les hormones, les stéroïdes, l'avidine, la biotine, les micro-organismes et les haptènes et les substances non biologiques susceptibles de se lier de façon spécifique avec un coordinat, telles que les médicaments. Des exemples d'analytes qui peuvent être détectés par le procédé de l'invention sont cités dans la demande de brevet EP 17 908 incorporée dans la présente description à titre de référence.

A titre d'exemples spécifiques d'analyte, on peut citer notamment : l'hormone adeno-corticotrope (ACTH), l'hormone antidiurétique (ADH), l'aldostérone, l'albumine, l'AMP cyclique, l'androsténedione, l'angiotensine, les anticorps antithyroglobuline, les antigènes carbohydrates : CA 125, CA 19-9 et CA 15-3, le cortisol, la digoxine, la digitoxine, l'estriol, la ferritine, la gastrine, l'hormone de croissance (HGH), l'hormone lactogène placentaire (HPL), l'insuline, le méthotréxate, la myoglobine, le parathyryne, le pepsinogène, la 17$\alpha$ hydroprogestérone, la thyroglobuline, la thyroxine binding globuline, le glucagon, la trypsine, l'hépatite : HBe et anti HBe, HBs et anti HBs, HBe et anti HBe, la particule delta et l'anti-particule delta, la transferrine, l'IgG, l'IgM, l'IgA, la C3, l'haptoglobuline, la ceruloplasmine, l'alpha 1 antitrypsine, le facteur rhumatoïde et plus particulièrement l'antigène carcicoembryonnaire (ACE), l'alpha foetoproteine (AFP), l'estradiol, la progestérone, la testostérone, l'hormone thyreostimulante (TSH), la triiodotryrosine (T3), la triiodothyrosine libre (FT3), la thyroxine (T4), la thyroxine libre (FT4), la prolactine, l'hormone luteinisante (LH), l'hormone folliculo stimulante (FSH), l'hormone thryreostimulante (TSH), les IgE totales.

Le composé luminescent mis en oeuvre dans le procédé de l'invention peut être choisi parmi les composés fluorescents, chimioluminescents ou phosphorescents.

Comme composé fluorescent on peut utiliser tous les composés qui absorbent la lumière à des longueurs d'onde supérieures à 300 nm, de préférence supérieures à 400 ou 450 nm et qui ont un coefficient d'extinction supérieur à $10^4$ au-dessus de 400 nm.

Une autre classe de composés fluorescents, qui conviennent aux fins de l'invention, est constituée par les chélates de terres rares fluorescents et les molécules organiques fluorescentes à durée de vie élevée, tels que les dérivés du pyrène.

Des exemples de composés fluorescents appropriés aux fins de l'invention sont cités notamment dans le brevet EP 15 695 et dans le brevet US 3 998 943 incorporés dans la présente description à titre de référence.

Les composés fluorescents particulièrement préférés sont la fluoresceine, la fluoresceine isothiocyanate, les chelates de terres rares et les cryptates de terres rares décrits par la demanderesse dans la demande de brevet FR 84 14 799.

On peut également utiliser dans le procédé selon l'invention un composé chimioluminescent, tels que le luminol et les esters d'acridinium (Methods in Enzymology 1978, 57, 424) ou des composés fluorescents, tels que la fluorescéine, excités par le produit de la réaction d'un oxalate avec de l'eau oxygénée (Acc. Chem. Res. 1969, 2, 80).

Les composés phosphorescents, tels que par exemple l'érythrosine et l'éosine (Biochem. J. 1979, 183, 50) conviennent également comme composés luminescents aux fins de l'invention.

On notera que l'érythrosine et l'éosine, qui sont des composés iodés, peuvent également être utilisés comme motifs contenant au moins un atome lourd.

Le couplage de l'un des réactifs avec le composé luminescent est réalisé selon des procédés classiques de couplage de manière à réaliser une liaison de covalence entre ledit réactif et le composé luminescent. On peut également fixer le composé luminescent sur l'un des réactifs par adsorption.

L'atome lourd présent sur l'un des réactifs peut être introduit par substitution directe, par exemple dans le cas des halogènes, par substitution sur des motifs, présents dans la molécule biologique, tels que les noyaux aromatiques ou par fixation sur le réactif de motifs contenant un atome lourd. La fixation de ces motifs peut être réalisé par tous les moyens de couplage couramment utilisés pour les protéines, par exemple par des agents chelatants ou par couplage par pont disulfure dans le cas du mercure comme cela est décrit dans le brevet GB 2 109 407. De préférence, l'atome lourd est un atome d'iode introduit dans le second réactif par iodation, par exemple selon le procédé BOLTON A.E., HUNTER W.N. [Biochem. J. 133, 529, 1973].

L'atome lourd ou les motifs contenant un atome lourd peuvent également être fixés sur l'un des réactifs par l'intermédiaire d'une molécule appropriée comportant à la fois des fonctions capables de se fixer sur ledit réactif et des fonctions capables de fixer l'atome lourd ou les motifs contenant un atome lourd. Par exemple, on peut utiliser comme molécule intermédiaire un polypeptide tel que la polylysine, les réactions de fixation sur le réactif et avec l'atome lourd ou les motifs contenant un atome lourd étant réalisés par des méthodes de couplage classiques.

L'utilisation d'une telle molécule intermédiaire permet à la fois d'augmenter le nombre d'atomes lourds par molécule de réactif sans affecter considérablement l'immunoréactivité dudit réactif.

A titre d'exemples de "motifs contenant au moins un atome lourd" on citera les dérivés iodés du succinimide, tels que les dérivés ci-après :

– N-[3-(3,5-iodo-4-hydroxyphényl) propionyloxy] succinimide ester ;
– N-[3-(3-iodo-4-hydroxyphényl) propionyloxy] succinimide ester ;
– N-[2-(4-iodophénylsulfonamido) acétoxy] succinimide ester ;
– N-[6-(4-iodophénylsulfonamido) hexyloxy] succinimide ester ;

ainsi que les produits de couplage de ces composés avec un polypeptide, tel que la polylysine.

Ces dérivés organiques iodés se combinent directement avec les réactifs mis en oeuvre dans le procédé de l'invention par mis en contact de ceux-ci avec une solution dudit réactif dans un tampon approprié.

L'addition des premier et second réactifs au cours du procédé de l'invention peut être simultanée ou séquencée. Dans le cas d'une addition séquencée on fait avantageusement incuber le milieu entre chaque addition de réactif.

L'excitation du milieu résultant est effectuée au cours de la dernière incubation ou après celle-ci suivant que la mesure est faite en cinétique ou à l'équilibre.

Cette excitation est réalisée à l'aide d'énergie lumineuse lorsque le composé luminescent est un composé fluorescent ou phosphorescent ou à l'aide de réactifs chimiques appropriés lorsque le composé luminescent est un composé chimioluminescent.

L'excitation par énergie lumineuse est réalisée à une longueur d'ondes dans le spectre d'absorption du composé fluorescent ou phosphorescent utilisé.

On indiquera que l'excitation lumineuse peut être faite de manière classique ou de façon pulsée, par exemple selon le procédé décrit par WIEDER dans le brevet US 4 058 732. Dans ce dernier cas, il y aura lieu de mettre en oeuvre un composé luminescent ayant une longue durée de décroissance luminescente (fluorescente ou phosphorescente) par rapport à la durée de décroissance luminescente des substances ambiantes, telles que notamment les substances contenues dans le milieu à doser et le matériel de dosage. De préférence cette durée de décroissance doit être supérieure à 1 microseconde. Bien entendu, la durée de l'excitation devra être inférieure à la durée de décroissance luminescente, du composé luminescent choisi. Avantageusement, on utilise comme composés fluorescents ayant une longue durée de décroissance fluorescente (ou une valeur élevée de 1/2 vie) les chélates de terres rares ou les cryptates de terres rares décrits par la demanderesse dans la demande de brevet FR 84 14799.

D'autre part, on notera que le procédé de l'invention est réalisé en phase liquide et que la mesure de la fluorescence ou la phosphorescence peut être effectuée après avoir déposé le milieu réactionnel sur une phase solide, par exemple, une bandelette, un gel ou tout autre support approprié.

Le procédé selon l'invention convient aussi bien pour les méthodes dites par excès que pour les méthodes dites par compétition.

Ainsi, dans le cas d'une méthode par excès, le procédé de l'invention consiste ;

1) à ajouter audit milieu contenant l'analyte recherché, un premier réactif constitué par un récepteur dudit analyte, couplé avec un composé luminescent ;
2) à ajouter un second réactif constitué par un ou plusieurs autres récepteurs dudit analyte, ledit second réactif étant couplé avec un atome lourd ou des motifs contenant un atome lourd,
3) à faire incuber le milieu résultant comme indiqué ci-dessus,
4) à exciter le milieu résultant, et
5) à mesurer le signal émis à l'équilibre ou en cinétique.

Dans le cas d'une méthode par compétition, le procédé de l'invention consiste :

1) à ajouter, audit milieu contenant l'analyte recherché, un premier réactif constitué par un récepteur dudit analyte, couplé avec un atome lourd ou des motifs contenant un atome lourd ;
2) à ajouter un second réactif constitué de l'analyte couplé avec un composé luminescent,
3) à incuber le milieu dans les conditions ci-dessus ;
4) à exciter le milieu résultant, et
5) à mesurer le signal émis à l'équilibre ou en cinétique.

Selon une autre variante de mise en oeuvre du procédé de l'invention dans le cas d'une méthode par compétition, on ajoute d'abord, au milieu contenant l'analyte recherché, un premier réactif constitué par un récepteur dudit analyte, ledit récepteur étant couplé avec un composé luminescent et on ajoute comme second réactif l'analyte sur lequel est couplé un atome lourd ou des motifs contenant un atome lourd, les étapes suivantes étant identiques à celles définies ci-dessus.

Le procédé selon l'invention s'applique particulièrement bien aux dosages immunologiques d'antigènes ou d'haptènes par excès ou par compétition.

Par exemple, dans le cas du dosage par compétition d'un antigène ou d'un haptène on utilise comme premier réactif un anticorps correspondant marqué à la fluorescéine ou iodé et une quantité donnée de l'antigène iodé ou marqué à la fluorescéine.

Dans le cas du dosage par excès, d'un antigène ou d'un haptène on utilise deux anticorps de spécificités différentes pour l'antigène ou l'haptène recherché, l'un étant marqué à la fluorescéine et l'autre iodé. Bien entendu dans ce type de dosage on peut également utiliser d'autres composés fluorescents et motifs comportant au moins un atome lourd, tels que ceux cités précédemment.

La présente invention a également pour objet un coffret pour dosage ou kit comprenant essentiellement :

– un premier réactif constitué d'au moins un récepteur de l'analyte à déterminer ;

– un second réactif constitué d'au moins un des éléments du produit de la réaction de l'analyte avec au moins l'un de ses récepteurs, l'un des réactifs étant couplé avec un composé luminescent et l'autre réactif étant couplé avec un atome lourd ou des motifs comprenant au moins un atome lourd.

– des échantillons standard contenant des quantités connues de l'analyte à déterminer pour établir les courbes standard ou gamme étalon.

– les diluants ou tampons nécessaires au dosage.

Lorsque le composé luminescent est un composé chimioluminescent, le coffret de dosage selon l'invention contient de plus les réactifs chimiques appropriés nécessaires à l'excitation.

L'invention va être maintenant décrite plus en détail par les exemples non limitatifs ci-après dans lesquels la substance à détecter ou à déterminer est un anticorps ou un antigène.

Dans ces exemples on a utilisé les composés ci-après :

– des gammaglobulines de lapin Miles ($\gamma_{GL}$) ;

– des gammaglobulines de mouton anti-lapin ($\gamma_{G\ MAL}$) obtenues par passage d'un antisérum de mouton immunisé avec des $\gamma_{GL}$ sur une colonne de DEAE-cellulose ;

– du sérum albumine humaine (HSA) à raison de 10 mg/ml dans un tampon phosphate 0,1 M pH 7,4 ;

– de la fluorescéine isothiocyanate isomère I ;

– de l'ester hydroxy succinique de l'acide 3-(4-hydroxyphényl)-propionique (NHSPP : réactif de Bolton et Hunter)

– du métabisulfite de sodium ;

– le 1,3,4,6-tétrachloro-3a,6a-diphényl-glycoluril comme agent iodogène ;

– DEAE-cellulose Whatman DE 52 comme échangeur d'ions ;

– une colonne de gel de filtration PD 10 Pharmacia ;

– un tampon phosphate (TP) ;

les mesures de fluoresecnce ont été effectuées sur PERKIN ELMER LS 5 fentes 2,5/10 nm, avec une excitation à 495 nm, une émission à 520 nm et un Fix à 15 ;

– les anticorps monoclonaux anti-prolactine $E_1$ et 3D3 contenus dans les trousses pour les dosages immunoradiométriques de la prolactine fabriquées par la compagnie ORIS INDUSTRIE SA et connues sous la dénomination commerciale "ELSA PROL",

– les anticorps monoclonaux anti-CEA G 12, G 13 et G 15 contenus dans les trousses pour les dosages immunoradiométriques de l'antigène carci no embryonnaire fabriquées par la compagnie ORIS INDUSTRIE SA et connues sous la dénomination commerciale "ELSA-CEA".

Exemple 1 : mise en évidence de l'effet d'atome lourd

a) Marquage de $\gamma_{GL}$ à la fluorescéine

On a dissous 1,44 mg du composé fluorescent (FITC) dans 1 ml d'eau et amené le pH à 9,5 avec de la soude.

On a dissous 24 mg de $\gamma_{GL}$ dans 2 ml de tampon phosphate 0,05 M pH 7,4 et on a ajouté 200 µl de la solution contenant le composé fluorescent. La réaction a été effectuée pendant 2 heures à température ambiante, le pH étant maintenu à 9,5 avec de la soude diluée.

Le mélange réactionnel a été alors dialysé une nuit contre un tampon phosphate 0,05 M, pH 7,4.

La solution a ensuite été passée sur une colonne de DEAE-cellulose Whatman DE 52 équilibrée avec du tampon phosphate 0,05 M pH 7,4.

Différentes fractions ont été éluées car gradient de salinité croissante (NaCl). Le rapport molaire fluorescéine/protéine ($\gamma_{GL}$) est déterminé par la formule de

$$F/P = \frac{A_{495}}{\varepsilon_{495}} \times \frac{\varepsilon_{280} \times 150\ 000}{A_{280} - 0{,}35\ A_{495}}$$

dans laquelle :

– $A_X$ est l'absorption à la longueur d'onde X ;
– ε représente le coefficient d'absorption molaire ;
– $\varepsilon_{495}$ = 72000
– $\varepsilon_{280}$ = 1,4 pour 0,1% poids/volume

Les différentes fractions obtenues en fonction de la salinité de la phase mobile sont indiquées ci-après :

| [NaCl] | F/P | Concentration approximative en $\gamma_{GL}$ |
|---|---|---|
| 0,05 M | $\simeq$ 1,3 | 120 µg/ml |
| 0,1 M | $\simeq$ 2,2 | 140 µg/ml |
| 0,2 M | $\simeq$ 3,5 | 240 µg/ml |
| 0,4 M | $\simeq$ 5,5 | 180 µg/ml |

b) Marquage de $\gamma_{G\ MAL}$ à l'iode

On a effectué ce marquage par la méthode utilisant la Chloramine T de R. HUNTER (Proc. Soc. Exp. Biol. Med. 133 (3) 989-1970). On a mis en contact pendant 3 min :

– 200 µl de $\gamma_{G\ MAL}$ à 3,5 mg/ml
– 100 µl de KI à $10^{-3}$ M dans de l'eau
– 200 µl de Chloramine T à $10^{-2}$ M dans de l'eau,

puis on a ajouté 200 µl d'une solution aqueuse $10^{-2}$ M de MBS.

La solution obtenue a été chargée sur une colonne PD 10 équilibrée en tampon phosphate 0,05 M, pH 7,5 (débit de pompe 16 ml/h). La détection en sortie de colonne a été effectuée par mesure de la densité optique à 280 nm. La fraction correspondant au sommet du premier pic a été recueillie ; sa concentration en $\gamma^{I}_{G\ MAL}$ a été évaluée à 0,24 mg/ml

c) Mise en évidence de l'effet de l'atome d'iode

On a préparé trois solutions :

- solution témoin : ( - 50 µl de TP 0,1 M pH 7,4
  ( - 200 µl de HSA
- solution libre : ( - 50 µl de TP 0,1 M pH 7,4
  ( - 50 µl d'une fraction de $\gamma_{GL}$ marqué à la fluorescéine ($\gamma^{F}_{GL}$) diluée 1/400 dans HSA
  ( - 150 µl de HSA
- solution liée ( - 50 µl de $\gamma_{G\ MAL}$ marqué à l'iode ($\gamma^{I}_{G\ MAL}$)
  ( - 50 µl de $\gamma^{F}_{G}$ diluées 1/400 dans HSA
  ( - 150 µl de HSA

On a fait incuber pendant 1 h 30 min et on a ajouté 250 µl de tampon phosphate 0,1 M pH 7,4 et on a mesuré la fluorescence pour une excitation à 495 nm.

On a déterminé l'efficacité E de la transition intersystème par la formule :

$$E = \frac{\text{I solution libre} - \text{I solution liée}}{\text{I solution libre} - \text{I solution témoin}}$$

dans laquelle I est l'intensité de fluorescence.

Les résultats obtenus avec la fraction F/P = 3,5 figurent dans le tableau I ci-après. Ils montrent que 16% de l'énergie d'émission de la molécule de fluorescéine ont été transférés de façon non radiative. Etant donné que la quantité de $\gamma_{G\ MAL}$ utilisée est une quantité en excès, on considère que toutes les $\gamma_{GL}$ sont liées.

Exemple 2 : utilisation de motifs polyiodés

Du NHSPP a été dissous dans un mélange benzène/acétaldéhyde 1 : 1 pour donner une solution de 1,3 $10^{-2}$ mol/l.

On a mis en contact :

| | | |
|---|---|---|
| - NHSPP évaporé au fond d'un tube | | 100 µl |
| - $\gamma_{G\ MAL}$ | 200 µl | 3 mg/ml |

On a laissé agir 15 min dans la glace puis on a ajouté :

| | |
|---|---|
| – KI ($5.10^{-1}$ M) | 20 µl |
| – Chloramine T ($5.10^{-2}$ M) | 20 µl |
| et après 1 min on a ajouté | 20 µl de MBS $5.10^{-2}$ M |

La solution a été chargée sur une colonne PD 10 ; le sommet du premier pic a été recueilli ; il présentait une densité optique à 280 nm de 0,527. On a mesuré la fluorescence pour une excitation a 495 nm des trois solutions préparées dans les mêmes conditions qu'à l'exemple 1.

Les résultats, qui figurent dans le tableau I ci-après, montrent que l'efficacité du procédé est accrue par l'utilisation de motifs polyiodés.

Exemple 3

a) Préparation du réactif iodé

On a mis en contact dans un tube les composés suivants :

| | |
|---|---|
| – NHSPP ($10^{-3}$ M) | 100 µl |
| – KI ($10^{-1}$ M) | 100 µl |
| – Chloramine ($10^{-2}$ M) | 100 µl |

Puis après 3 min de contact on a ajouté 100 µl de MBS ($10^{-2}$ M).

On a ensuite effectué une extraction avec 2 fois 2 ml de benzène contenant 20 µl de diméthylformamide. La phase organique a été évaporée dans un autre tube et on a ajouté 100 µl de $\gamma_{G\ MAL}$. La réaction a été effectuée pendant 15 min dans de la glace. On a déposé sur une colonne PD 10. Le sommet du pic recueilli avait une densité optique de 0,1 à 280 nm.

b) Mise en évidence de l'effet atome lourd

On a utilisé cette fraction de $\gamma^{I}_{G\ MAL}$ pour réaliser une solution liée dans les mêmes proportions qu'à l'exemple 1 en utilisant la fraction $\gamma^{F}_{G}$ 1/400 F/P = 3,5.

On a par ailleurs préparé les solutions témoin et libre dans les mêmes conditions qu'à l'exemple 1. Les résultats de mesure de fluorescence figurent dans le tableau I.

Exemple 4

On a mis en contact :

- NHSPP $10^{-2}$M évaporé 100 µl
- iodogène dans $CCl_4$ 5 $10^{-2}$M évaporé 20 µl
- KI 5 $10^{-2}$M 20 µl
- tampon phosphate 0,05 M, pH 7,4 40 µl

Après 1 min de contact on a extrait 2 fois avec 1 ml de benzène dans du diméthylformamide (1%). La phase organique a été évaporée dans un autre tube et on a ajouté 10 µl de $\gamma_{G\ MAL}$, puis on a laissé agir 15 min dans de la glace. On a séparé les $\gamma^{I}_{G\ MAL}$ formées par dialyse contre 1 l de tampon phosphate 0,05 M pH 7,4.

On a utilisé une dilution au 1/10 de $\gamma^{I}_{G\ MAL}$ pour former une solution libre et une solution liée avec les $\gamma^{F}_{GL}$ 1/400 F/P = 3,5 préparées selon l'exemple 1.

Les résultats obtenus sont reportés dans le tableau I.

TABLEAU I

| Exemples n° \ Solutions testées | Solution témoin | Solution libre | Solution liée | Efficacité |
|---|---|---|---|---|
| 1 | 43,5 | 77,9 | 72,4 | 0,16 |
| 2 | 36,7 | 76,6 | 65,2 | 0,286 |
| 3 | 34,4 | 59,4 | 53,9 | 0,21 |
| 4 | 33 | 64,5 | 56,5 | 0,25 |

Exemple 5

On a évalué l'effet de marquage des $\gamma_{GL}$ avec la fluorescéine. On a utilisé les $\gamma_{G\ MAL}$ marquées à l'iode selon l'exemple 4 et diluées au 1/20 dans HSA et les différentes fractions $\gamma^{F}_{GL}$ préparées à l' exemple 1.

Les mesures de fluorescence ont été effectuées selon le mode opératoire de l'exemple 1. Les résultats obtenus sont les suivants :

| Fraction $\gamma_{GL}^{F}$ F/P | Solution liée | Solution libre | E |
|---|---|---|---|
| 1,3 | 5,4 | 6,5 | 0,17 |
| 2,2 | 11,7 | 14,7 | 0,20 |
| 3,5 | 26,3 | 35,2 | 0,25 |
| 5,5 | 26,8 | 47,7 | 0,44 |

La fluorescence de la solution témoin était de 26,8.

On remarque que plus le nombre de fluorescéine par $\gamma_{GL}$ est élevé plus l'effet est important. Cet accroissement est certainement lié à la délocalisation de l'énergie de l'état excité de la fluorescéine par transfert intermoléculaire entre molécules de fluorescéine.

Exemple 6

On a établi la courbe de dilution de l'anticorps $\gamma_{G\ MAL}$ marqué selon le mode opératoire de l'exemple 4 avec les $\gamma_{GL}^{F}$ F/P = 5,5 diluées au 1/400.

Les résultats obtenus sont les suivants :

| Dilution de $\gamma_{G\ MAL}$ dans HSA | I | E % |
|---|---|---|
| 1/10 | 26,6 | 44 |
| 1/20 | 26,7 | 44 |
| 1/100 | 43,2 | 9,4 |
| 1/500 | 46,4 | 2,7 |
| 1/1000 | 47,7 | 0 |

On remarque que l'efficacité décroît lorsque le facteur de dilution croît.

Exemple 7 : mise en oeuvre du procédé de l'invention dans une méthode de dosage par excès.

a) Marquage de l'anticorps monoclonal anti-prolactine $E_1$ à la fluorescéine.

On a mélangé 0,5 ml d'une solution de $E_1$ à 9,7 mg/ml et 0,2 mg de FITC (Molecular Probe) dans 0,5 ml d'eau. Le pH a été ajusté à 9,3 avec de la soude. On a laissé réagir 3 h à température ambiante tout en maintenant le pH constant. La solution a ensuite été dialysée pendant 20 h après neutralisation à pH 7, contre 2 fois 2 l de tampon phosphate 0,05 M pH 7,4.

On a réalisé une colonne d'environ 15 ml de gel DEAE-cellulose équilibrée en tampon phosphate 0,05 M pH 7,4. La colonne a été chargée avec le milieu réactionnel dialysé et on a élué avec des tampons contenant respectivement 0,05 M ; 0,1 M ; 0,2 M ; 0,4 M ; 0,7 M et 1 M de NaCl ; pH 7,4.

Les pics élués avec les tampons 0,4 M NaCl et 0,7 M NaCl ont été recueillis et dialysés.

Pour le pic obtenu avec le tampon à 0,4 M NaCl on a noté une densité optique à 280 nm de 0,184 et une densité optique à 495 nm de 0,167, ce qui correspond à une concentration approximative d'anticorps de 90 µg/ml et à un rapport F/P d'environ 4.

b) Marquage de l'anticorps monoclonal 3D3 à l'iode.

Le protocole est identique à celui de l'exemple 4. Les produits utilisés sont les suivants :

| | | |
|---|---|---|
| - NHSPP | ($10^{-2}$M) | 200 µl |
| - Iodogène | (5 $10^{-2}$M) | 40 µl |
| - KI | (5 $10^{-2}$M) | 40 µl |
| - tampon phosphate 0,05M pH 7,4 | | 40 µl |

L'extraction a été faite 2 fois avec 1 ml de benzène à 1% dans le diméthylformamide. Après évaporation on a ajouté 200 µl de 3D3 à 3,3 mg/ml et on a laissé réagir 15 min dans de la glace. On a séparé sur colonne de PD 10 et on a récupéré la fraction ayant une densité optique de 0,485 à 280 nm, qui avait une concentration de 350 µg/ml.

c) Dosage par excès.

- $E_1$ fluorescent ($E_1^F$) à 3 µg/ml dans une solution de HSA à 5 mg/ml ;
- 3D3 marqué à l'iode $3D3^I$ à 110 µg/ml dans la même solution de HSA ;
- 3D3 à 110 µg/ml dans la même solution de HSA ;
- Prolactine PRL 0,6 µg/ml dans la même solution de HSA.

On a réalisé les trois solutions suivantes :

- Solution témoin : 150 µl de HSA à 5 mg/ml.
- Solution liée $^I$ : ( 50 µl de $E_1^F$ ( 50 µl de $3D3^I$ ( 50 µl de PRL.
- Solution liée : ( 50 µl de $E_1^F$ ( 50 µl de 3D3 ( 50 µl de PRL

On a laissé incuber chaque solution 2 h à la température ambiante et on a ajouté 100 µl de HSA et 250 µl de tampon phosphate.

Les mesures de fluorescence effectuées selon le mode opératoire de l'exemple 1 ont donné les résultats suivants :

| | Fluorescence |
|---|---|
| solution témoin | 29,7 |
| solution liée I | 197,3 |
| solution liée | 231,1 |
| Δ | 33,8 |
| E | 0,17 |

On observe donc le même phénomène dans une méthode par excès (exemple 7) en marquant deux anticorps de spécificités différentes et dans une méthode par compétition (exemples 1 à 6).

Exemple 8 : étude cinétique.

On a répété le dosage de l'exemple 7 à la température ambiante en utilisant une solution de prolactine PRL à 0,6 µg/ml dans 100 µl de HSA à 5 µg/ml et les mêmes réactifs $E_1^F$ et $3D3^I$ mais en faisant varier leur concentration et on a mesuré l'efficacité E de la transition intersystème en fonction du temps d'incubation.

Les concentrations utilisées étaient les suivantes :

$E_1^F$ à 3 µg/ml dans une solution de HSA à 5 µg/ml
$Ac^I = 3D3^I$ à 360 µg/ml dans une solution de HSA à 5 µg/ml.

$E_{1/2}^F$ à 1,5 µg/ml dans la même solution de HSA
$Ac^I = 3D3^I$ à 360 µg/ml dans la même solution de HSA

$E_{1/4}^F$ à 0,75 µg/ml dans la même solution de HSA
$Ac_{1/3}^I = 3D3^I$ à 120 µg/ml dans la même solution de HSA

$E_{1/2}^F$ à 1,5 µg/ml dans la même solution de HSA
$Ac_{1/3}^I$ à 120 µg/ml dans la même solution de HSA

Les résultats obtenus sont portés sur le graphe de la figure 1 annexée sur lequel, on a porté en abscisses le temps d'incubation en minutes et en ordonnées l'efficacité E en %.

Ces résultats montrent que le procédé de l'invention peut également être utilisé en cinétique.

Exemple 9 : courbe standard

On a préparé six solutions de PRL à 600, 150, 75, 30, 7,5 et 0 ng/ml dans une solution de HSA à 5 mg/ml.

On a fait incuber 50 µl de chaque solution dans un tube avec 50 µl de $E_1^F$ à 3 µg/ml et 50 µl de $3D3^I$ à 120 µg/ml pendant 30 min à la température ambiante et on a ajouté 350 µl de tampon phosphate. On a mesuré ensuite l'efficacité d'inhibition E en fonction de la valeur du milieu standard constitué par 150 µl de HSA à 5 mg/ml sachant que 1 µU = 30 ng.

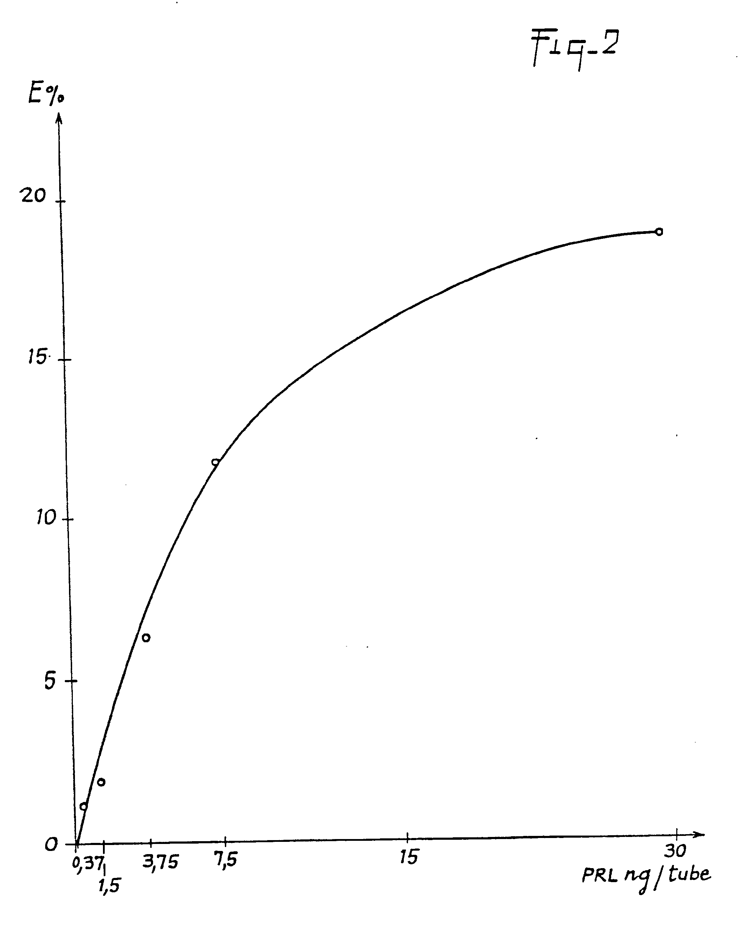

Les résultats obtenus sont portés sur le graphe de la figure 2 annexée sur lequel on a porté en abscisses la concentration en prolactine PRL exprimée en ng/tube et en ordonnées l'efficacité E en %.

On peut ainsi établir des courbes standards pour chaque protéine à doser.

Exemple 10

Dans cet exemple, on a utilisé les anticorps monoclonaux anti-CEA G 12, G 13 et G 15.

a) marquage de l'anticorps anti-CEA G 12 à la fluorescéine

On a utilisé le même protocole qu'à l'exemple 7a. La dialyse et l'élution de la colonne ont eté éffectuées avec le tampon TRIS 0,01 M pH 8. On a récupéré les pics élués avec 0,2 et 0,4 M NaCl. La valeur F/P était d'environ 2,75 pour le pic élué à 0,2 M. La solution recueillie a été concentrée à 800 µg/ml.

b) marquage de G 15 à l'iode

Le protocole est identique à celui de l'exemple 4. Les produits utilisés sont les suivants :

| | |
|---|---|
| NHSPP ($10^{-2}$M) | 200 µl |
| iodogène (5 $10^{-2}$M) | 40 µl |
| KI (5 $10^{-2}$M) | 40 µl |
| tampon phosphate 0,05 M pH 7,4 | 40 µl |
| G 15 (5 mg/ml) | 200 µl |

La fraction ayant une densité optique de 0,84 à 280 nm a été recueillie ; sa concentration était de 600 µg/ml.

c) marquage de G 13 à l'iode

On a opéré comme ci-dessus et on a recueilli la fraction ayant une densité optique de 0,35 à 280 nm ; sa concentration était de 250 µg/ml.

On a utilisé un standard à 300 ng de CEA par ml.

$G_{12}^{F}$ a été dilué au 1/2000 dans HSA 5 mg/ml

$G_{15}^{I}$ a été dilué au 1/6 dans HSA

On a réalisé les trois solutions ci-après :

- solution témoin : 100 µl de HSA + 100 µl tampon
- solution liée : (50 µl de $G_{12}^{F}$ au 1/2000
  (
  (50 µl de $G_{15}^{I}$ 1/6
  (
  (100 µl du standard
- solution libre : 50 µl de $G_{12}^{F}$ au 1/2000
  50 µl de $G_{15}^{I}$ au 1/6
  100 µl de tampon

On a fait incuber ces solutions deux fois 1 h à 45°C et on a ajouté 300 µl de tampon phosphate.

On a mesuré la fluorescence de chaque solution pour une excitation à 495 nm et on a obtenu les résultats ci-après :

| Solutions | Intensité de fluorescence | efficacité |
|---|---|---|
| solution témoin : | 44,5 | E = 0,16 |
| solution liée : | 90,5 | ΔF = 8,8 |
| solution libre : | 99,3 | |

On a donc également une inhibition de 16% quand deux anticorps de spécificité différente sont liés à l'antigène.

On a réalisé le même essai en ajoutant, lors d'une troisième incubation, l'anticorps $G^{I}_{13}$ de spécificité différente de celle de G 12 et G 15.

Les solutions testées sont les suivantes :

| | |
|---|---|
| Solution liée | 50 µl de $G^{F}_{12}$ au 1/2000<br>50 µl de $G^{I}_{15}$ au 1/6<br>50 µl de $G^{I}_{13}$ au 1/3<br>100 µl du standard |
| Solution libre | 50 µl de $G^{F}_{12}$ au 1/2000<br>50 µl de $G^{I}_{15}$ au 1/6<br>50 µl de $G^{I}_{13}$ au 1/3<br>100 µl de tampon |

On a fait incuber ces solutions 3 fois pendant 1 h à 45°C et on a ajouté 250 µl de tampon phosphate. On a obtenu les résultats ci-après :

| Solution | Intensité de fluorescence | efficacité |
|---|---|---|
| solution témoin | 81 | 0,41 |
| solution liée | 106,7 | |
| solution libre | 124,9 | ΔF = 18,2 |

Ces résultats montrent que la présence d'un deuxième anticorps iodé sur un autre site de l'antigène augmente l'inhibition de la fluorescence de l'anticorps G 12 marqué à la fluorescéine.

Exemple 11 : courbe standard

On a préparé quatre solutions de CEA à 300, 200, 100 ng/ml et 0 ng/ml. On a fait incuber chaque solution avec $G^{F}_{12}$ dilué au 1/3000 dans HSA et $G^{I}_{13}$ et $G^{I}_{15}$, dilués respectivement au 1/3 et 1/6 dans une solution de HSA, et on a mesuré la fluorescence.

On a obtenu les résultats ci-après :

| Solution | Intensité de fluorescence | ΔF | E % |
|---|---|---|---|
| solution témoin | 54 | – | – |
| solution à 300 ng/ml | 65,9 | 11,9 | 0,5 |
| " à 200 ng/ml | 68 | 9,8 | 0,41 |
| " à 100 ng/ml | 71,8 | 6 | 0,25 |
| " à 0 ng/ml | 77,8 | 0 | 0 |

Exemple 12 : utilisation de dérivés iodés du succinimide comme motifs contenant au moins un atome lourd

A – préparation des dérivés iodés du succinimide

a) composé 1 : N-[3-(3,5-iodo-4-hydroxyphényl) propionyloxy] succinimide ester de formule :

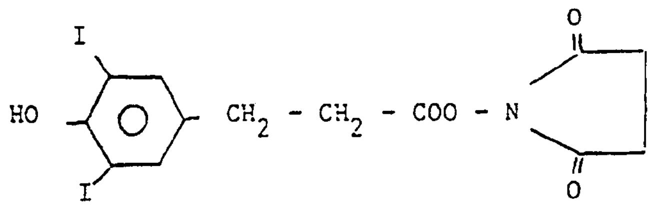

On a dissous $1.10^{-4}$ moles (26,3 mg) de N-[3-(4-hydroxyphényl) propionyloxy] succinimide ester (origine Fluka) dans 2,5 ml d'un mélange benzène/acétate d'éthyle (50/50 en V/V), puis on a ajouté en une fois $2.10^{-4}$ moles (86,4 mg) d'Iodogen® (origine Sigma), puis 83 mg d'iodure de potassium en solution dans 100 µl de tampon phosphate 0,05 M (pH 7,4) ; une intense coloration violette s'est développée instantanément. La réaction a été poursuivie à 20°C sous agitation pendant 15 min. (sous argon). La réaction a alors été stoppée par addition d'une solution saturée de métabisulfite de sodium dans de l'eau jusqu'à décoloration du mélange réactionnel. Les phases organiques ont été séparées par décantation, séchées sur $MgSO_4$ anhydre et évaporées sous vide. Le résidu a été repris dans du $CH_2Cl_2$ ou du benzène anhydre.

Le produit a alors été purifié par chromatographie sur colonne de gel de silice. L'éluant était un gradient discontinu de benzène/acétate d'éthyle. Le produit attendu a été élué par un mélange benzène/acétate d'éthyle 90/10 (V/V). La pureté du composé 1 a été contrôlée par C.C.M. (éluant : toluène 1/acétate d'éthyle 1 V/V) et comparée par C.C.M. (éluant : toluène 1/acétate d'éthyle 1 et la spectrométrie de masse étaient conformes avec la structure du produit. Rendement : 58%.

b) composé 2 : N-[3-(3-iodo-4-hydroxyphényl) propionyloxy] succinimide ester de formule :

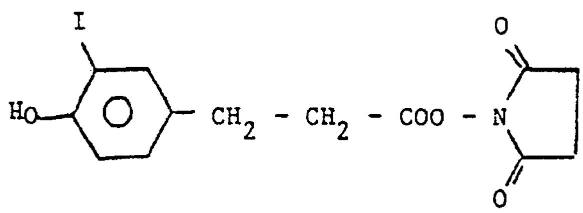

On a opéré selon le mode opératoire décrit ci-dessus pour le composé 1 en utilisant les ingrédients suivants dans les proportions indiquées ci-après :

- N-[3-(4-hydroxyphényl) propionyloxy] succinimide ester: $2.10^{-3}$ moles (0,526 g)
- Iodogen R (Sigma) : $2.10^{-3}$ moles (0,864 g)
- KI : $4.10^{-3}$ moles (0,584 g) dans 500 µl de tampon phosphate 0,05 M (pH 7,4)

Le produit obtenu a été soumis aux mêmes étapes de purification et de contrôle que le composé 1.

c) composé 3 : N-[2-(4-iodophénylsulfonamido) acétoxy] succinimide ester de formule :

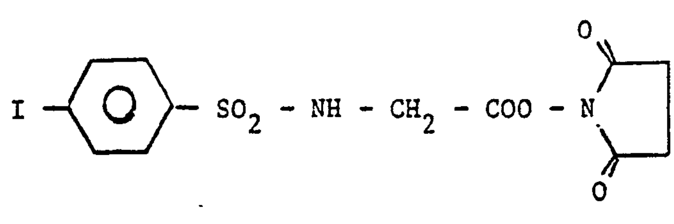

La synthèse de ce composé 3 a été effectuée en deux étapes distinctes après purification et isolement du produit intermédiaire A selon le schéma réactionnel ci-après :

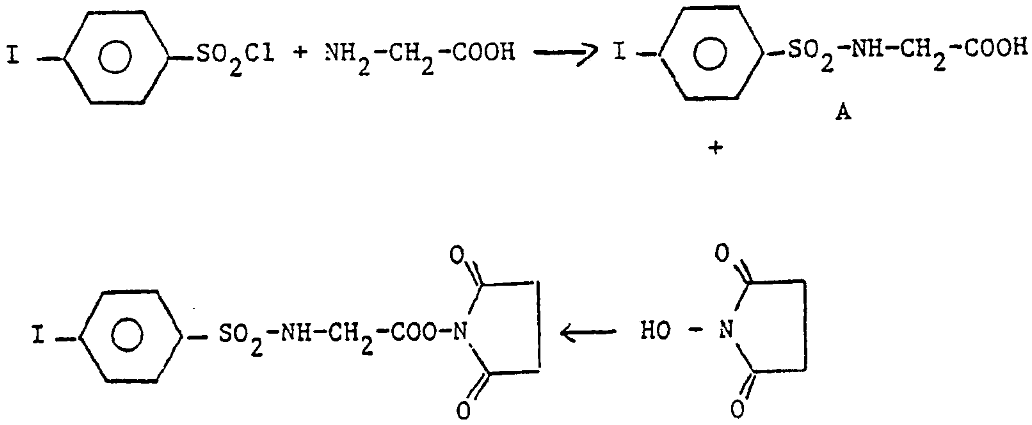

composé 3

1) synthèse de A :

$8.10^{-3}$ moles (2,4 g) de p-iodophénylsulfochlorure en solution dans 5 ml de dioxanne ont été ajoutés goutte à goutte à $7.10^{-3}$ moles (0,525 g) de glycocolle en solution aqueuse préalablement ajustée par de la soude 1 M (10 ml) à pH 9 et refroidie en bain de glace. Lorsque l'addition est terminée le bain de glace est retiré et la réaction est poursuivie pendant une heure à 20°C sous agitation. (Le contrôle du pH a été effectué au pH mètre et réajusté à pH 9 pendant toute la durée de la réaction).

En fin de réaction, le mélange a été dilué avec deux fois son volume d'eau distillée. La solution a été débarassée des insolubles de réaction par filtration. Le filtrat a été acidifié goutte à goutte sous agitation à pH 2 par de l'acide chlorhydrique 6 N. Le produit de condensation attendu a précipité abondamment en paillettes blanches. Le produit brut a été filtré, puis lavé par de l'eau distillée plusieurs fois, essoré et séché sous vide au dessicateur en présence de $P_2O_5$ pendant une nuit. Rendement : 73% (1,75 g).

La pureté du produit a été contrôlée par C.C.M. et les méthodes spectrophotométriques usuelles.

2) synthèse du composé 3 :

A une solution de 2 mmoles de A (0,682 g) et de 2 mmoles de N-hydroxysuccinimide (0,230 g) dans 10 ml de THF, préalablement refroidie à 0°C, on a ajouté en une fois 2 mmoles de D.C.C. (0,412 g). Le mélange réactionnel a été agité et maintenu une heure à cette température. Au bout de ce temps, le bain de refroidissement a été enlevé et on a filtré le mélange réactionnel sur verre fritté N° 3 ; le filtrat a été évaporé sous vide. Le résidu constitué d'une mousse blanche a été repris par du $CH_2Cl_2$, filtré sur célite®, puis réévaporé sous vide. Le produit brut ainsi obtenu a été recristallisé dans $CH_2Cl_2$. Poids obtenu : 0,371 g Rendement : 42%.

L'identité du produit a été contrôlée par les méthodes spectrophotométriques usuelles et sa composition confirmée par l'analyse centésimale.

(D.C.C. = dicyclohexyldiimide).

d) composé 4 : N-[6-(4-iodophénylsulfonamido) hexyloxy] succinimide ester de formule :

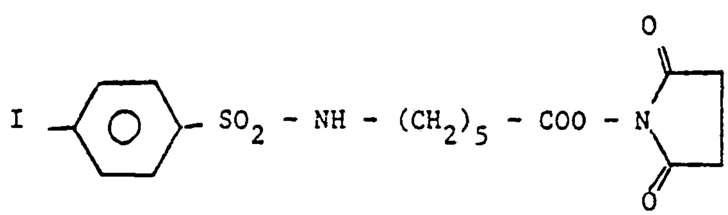

La synthèse du composé 4 a été effectuée en deux étapes après isolement du produit intermédiaire B et selon le schéma réactionnel suivant :

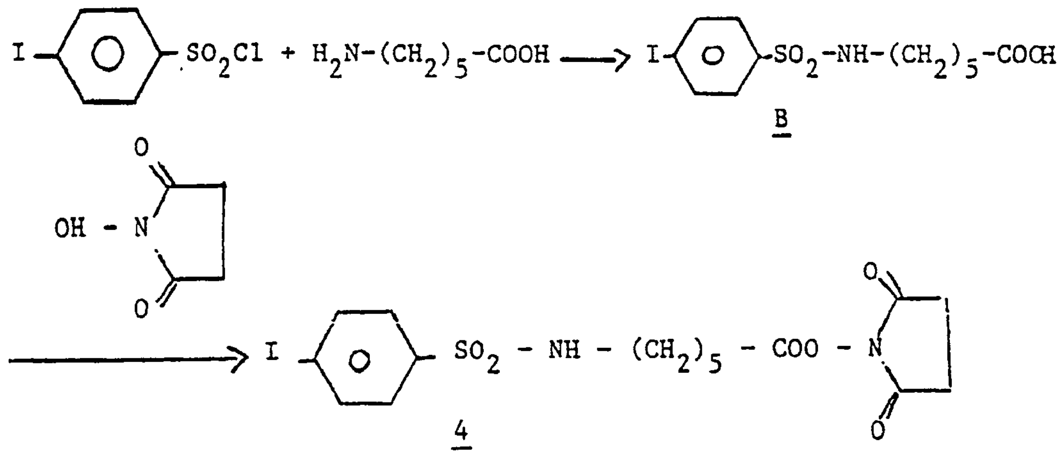

B

4

1) synthèse de B :

5 mmoles (1,52 g) de p-iodophénylsufochlorure en solution dans 5 ml de dioxanne ont été ajoutés goutte à goutte à une solution aqueuse de 7 mmoles (0,918 g) d'acide amino-5 caproïque (Fluka), préalablement ajustée à pH 9 par 10 ml de NaOH 1 M et refroidie au bain de glace.

L'addition du réactif iodé terminée, le bain de glace a été retiré et la réaction poursuivie pendant 3 heures à 20°C. (Le contrôle du pH a été également effectué durant toute la durée de la réaction comme dans l'exemple précédent).

Le mélange réactionnel a alors été filtré sur verre fritté N°3, et le filtrat acidifié à pH 4 par quelques gouttes d'HCl 12 N. Le produit B attendu a précipité alors abondamment. Celui-ci a été filtré, lavé abondamment sur le filtre avec de l'eau distillée, essoré, et enfin séché au dessicateur sous vide, sur $P_2O_5$ pendant une nuit. Poids obtenu : 1,30 g ; rendement 68%. La pureté du produit a été contrôlée par C.C.M. (silice) éluant : $CHCl_3$/méthanol (3/1 V/V). Son point de fusion était de : 154 ± 1°C. La structure du produit a été confirmée par I.R. L'analyse centésimale a confirmée la structure du produit.

3) synthèse du composé 4 :

On a opéré selon le mode opératoire décrit pour le composé 3 en utilisant les ingrédients ci-après :

| | |
|---|---|
| – produit B : | 2 mmoles (0,762 g) |
| – N-hydroxysuccinimide : | 3 mmoles (0,345 g) |
| – D.C.C. : | 3 mmoles (0,614 g) |
| – solvant THF : | 10 ml. |

Le temps de réaction a été de 1 heure à 4°C et une nuit à 20°C.

La purification du produit a été identique à celle du composé 3. Poids obtenu : 0,870 g ; rendement : 91%. La pureté a été contrôlée par C.C.M. (éluant acétate d'éthyle/$CH_2Cl_2$ (1 : 1 V/V) et par l'analyse centésimale. La structure du produit a été déterminée par spectrophotométrie IR et spectrométrie de masse. Celle-ci est conforme à la structure attendue pour le composé 4 décrit.

B – Couplage des dérivés de succinimide avec des anticorps

Dans cet exemple on a utilisé l'anticorps $3D_3$ (anticorps anti-prolactine).

$1.10^{-6}$ mole de $3D_3$ (10 mg/ml) en solution dans 200 µl de tampon phosphate 0,05 M (pH 7,4) et 200 µl de tampon borate 0,01 M (pH 9) a été ajouté à 2 mg du réactif diodé 1 (composé 1) qui a été préalablement dissous dans du $CH_2Cl_2$ et évaporé sous argon de manière à créer un coating sur les parois du tube de réaction. La réaction de couplage a été poursuivie sous agitation à 20°C pendant une heure. Au bout de ce temps, $3D_3$ marqué à l'iode a alors été purifié par chromatographie sur une colonne de PD 10 et le pic correspondant à l'anticorps marqué (volume mort de la colonne) a été isolé. La concentration a alors été mesurée par son absorbance à 280 nm.

L'anticorps marqué ainsi isolé a été utilisé à la concentration de 150 µg/ml dans le test d'inhibition de la fluorescence.

On a opéré de la même façon avec les composés 3 et 4.

De la même manière, on a couplé le composé 2 avec l'anticorps anti-CEA G 15. A cet effet, l'anticorps G 15 (200 µl à 6,5 mg/ml) a été mis en contact avec 200 µl du composé 2 (1,24 mg dans 3,75 ml de $CH_2Cl_2$ puis 200 µl évaporé au fond du tube) et 200 µl de tampon borate pH 9. On a laissé agir pendant 1 h 30 min à la température ambiante.

C – dosage par excès de la prolactine

Les réactifs utilisés sont les suivants :

– anticorps $3D_3^I$ : 150 µg/ml par dilution dans HSA à 5 g/l

– anticorps $E_I^F$ dilué à 1/100° µg/ml) en solution dans des gammaglobulines de lapin à la concentration de 5 mg/ml

– antigène prolactine (fournie par Immunotech) : 0,5 µg/ml en solution dans HSA à 5 g/l

– solutions diluantes :

- gammaglobulines de lapin (concentration 5 g/l) en solution dans le tampon phosphate 50 mM (pH 7,4)
- HSA (concentration 5 g/l) dans le tampon phosphate 50 mM (pH 7,4) ;
- témoin : HSA (5 g/l) + solution éluante de la purification (50 : 50 V/V)

On a préparé les trois solutions suivantes :

| | |
|---|---|
| – solution témoin : | 50 µl de $\gamma_{GL}$ |
| | 50 µl de HSA |
| | 50 µl de témoin |
| – solution liée : | 50 µl de $E_1^F$ |
| | 50 µl de prolactine |
| | 50 µl de $3D_3^I$ |
| – solution libre : | 50 µl de $E_1^F$ |
| | 50 µl de HSA |
| | 50 µl de $3D_3^I$ |

On a incubé chaque solution pendant une heure à 20°C. On a ensuite ajouté à chacune d'elles, avant lecture, 350 µl de tampon phosphate 0,05 M (pH 7,4).

Les mesures de fluorescence ont été effectuées à 496 nm (excitation) et 520 nm (émission) au fluorimètre et on a déterminé l'efficacité E.

Les résultats obtenus sont rassemblés dans le tableau suivant.

D – dosage par excès de l'antigène CEA

On a répété le iode opératoire ci-dessus en utilsant l'antigène CEA à la place de la prolactine et les réactifs ci-après :

réactif iodé : solution obtenue en B avec le composé 2. Cette solution a été amenée à 0,10 mg/ml après purification sur PD 10.

réactif fluorescent : solution au 1/2000 d'anticorps G 12 marqué à la fluoresceine.

L'incubation a été effectuée pendant 2 heures à 45°C. Les résultats obtenus figurent également dans le tableau ci-après.

| Dosage de | Dérivé iodé | Dérivé iodé / $3D_3$ en mole/mole | pH réaction couplage | E % |
|---|---|---|---|---|
| PROLACTINE | Composé 1 | 10/1 | 9,0 | 11 |
| | | 50/1 | 9,0 | 18 |
| | | 300/1 | 9,0 | 16,5 |
| | Composé 3 | 30/1 | 9,0 | 8,2 |
| | | 100/1 | 9,0 | 9 |
| | Composé 4 | 30/1 | 7,4 | 2,3 |
| | | 100/1 | 7,4 | 5 |
| | | 100/1 | 9,0 | 7 |
| CEA | Composé 2 | 20/1 | 9,0 | 11 |

Exemple 13 : Utilisation du produit de couplage d'un polypeptide et d'une molécule organique iodée comme motifs contenant au moins un atome lourd.

Dans cet exemple, on a préparé le produit de couplage de la polylysine avec le composé diiodé n° 1, dénommé ci-après réactif A. Ce réactif A peut être représenté par la forme statique ci-après :

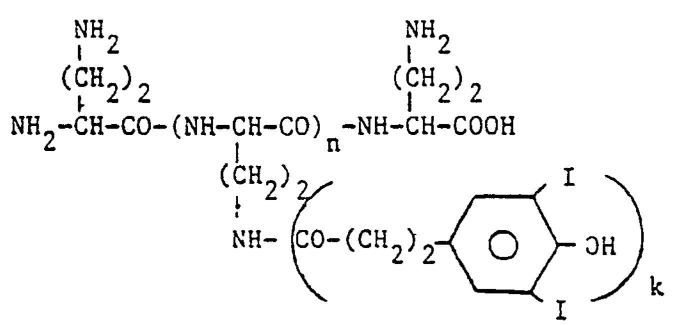

avec n = 327

k = 50

A – Synthèse du réactif A

5 mg de chlorhydrate de polylysine (P.M. 48000) fournie par Sigma Chemicals ont été dissous dans 500 µl de tampon borate (0.01 M en borate) pH 8,9, ajouté à pH 12 par de l'hydroxyde de sodium 0,5 M et ajoutés à 2,7 mg ($5.2 \times 10^{-6}$ moles) du composé 1 ci-dessus préalablement évaporé sur le fond d'un tube à essai à

partir d'une solution dans $CH_2Cl_2$. La réaction a été poursuivie pendant une heure à 20°C sous agitation.

Le produit de la réaction est élué sur PD 10 (Séphadex G 25) l'éluant étant du tampon phosphate (50 nM en phosphate) ; les fonctions ont été concentrées par centrifugation sur cônes Amicon 25 jusqu"à un volume de 90 µl.

B – Couplage du réactif A avec l'anticorps 3D3

A 80 µl du réactif A on a ajouté 120 µl d'un tampon phosphate 25 mM pH 5.

On a ajouté alors 100 µl d'une solution de carbodiimide à 2 mg/ml d'$H_2O$.

On a attendu 2 à 3 minutes, puis on a ajouté alors 400 µl d'une solution de l'anticorps $3D_3$ (200 µl de $3D_3$ à 9,6 mg/ml dans un tampon phosphate 50 mM pH 7,4 + 200 µl de tampon phosphate 200 mM pH 8).

Le mélange a été laissé 1 heure à 20°C puis 1 nuit à 4°C et encore 1 heure à 20°C.

La séparation s'est effectuée sur PD 10 en utilisant du tampon phosphate 50 mM pH 7,4 comme éluant.

On a recueilli dans le volume mort de la colonne une solution de 1 ml dont la densité optique à 280 nm était de 0,742. On a estimé la concentration en anticorps à 530 µg/ml et on a soumis cette fraction au test d'inhibition de la fluorescence selon le mode opératoire décrit à l'exemple 12.

Les intensités de fluorescence des différentes solutions obtenues étaient respectivement de :

Solution témoin : 16

Solution liée : 134

Solution libre : 150

L'efficacité E était donc de : 11,9 %.

**Revendications**

1. Procédé homogène de détection et/ou de détermination par luminescence d'un analyte dans un milieu susceptible de le contenir, par mise en évidence du produit de la réaction entre l'analyte et un récepteur correspondant, caractérisé en ce qu'il consiste :

1) à ajouter audit milieu un premier réactif constitué d'un récepteur dudit analyte ;

2) à ajouter un second réactif constitué d'au moins un des éléments du produit de la réaction de l'analyte avec au moins l'un de ses récepteurs, l'un des deux réactifs étant couplé avec un composé luminescent et l'autre réactif étant couplé avec un atome lourd ou des motifs contenant un atome lourd ;

3) à faire incuber le milieu résultant soit après l'addition de chaque réactif, soit après l'addition des deux réactifs ;

4) à exciter le milieu résultant, et

5) à mesurer, à l'équilibre ou en cinétique, le signal émis par le composé luminescent, ledit signal étant modulé par l'effet d'atome lourd.

2. Procédé selon la revendication 1 consistant en une méthode par excès, caractérisé en ce qu'il consiste:

1) à ajouter audit milieu contenant l'analyte recherché, un premier réactif constitué par un récepteur dudit analyte, couplé avec un composé luminescent ;

2) à ajouter un second réactif constitué par un ou plusieurs autres récepteurs dudit analyte, ledit second réactif étant couplé avec un atome lourd ou des motifs contenant un atome lourd ;

3) à faire incuber ledit milieu après chaque addition de réactifs ou après l'addition des deux réactifs,

4) à exciter le milieu résultant, et

5) à mesurer le signal émis à l'équilibre ou en cinétique.

3. Procédé selon la revendication 1 consistant en une méthode par compétition, caractérisé en ce qu'il consiste :

1) à ajouter audit milieu contenant l'analyte recherché, un premier réactif constitué par un récepteur dudit analyte sur lequel est couplé un atome lourd ou des motifs contenant un atome lourd ;

2) à ajouter un second réactif constitué de l'analyte couplé avec un composé luminescent,

3) à faire incuber ledit milieu après chaque addition de réactifs ou après l'addition des deux réactifs,

4) à exciter le milieu résultant, et

5) à mesurer le signal émis à l'équilibre ou en cinétique.

4. Procédé selon la revendication 1 consistant en une méthode par compétition, caractérisé en ce qu'il

consiste :

1) à ajouter audit milieu contenant l'analyte recherché, un premier réactif constitué par un récepteur dudit analyte, ledit récepteur étant couplé avec un composé luminescent,

2) à ajouter, à titre de second réactif, l'analyte sur lequel est couplé un atome lourd ou des motifs contenant un atome lourd,

3) à faire incuber ledit milieu soit après l'addition de chaque réactif soit après l'addition des deux réactifs,

4) à exciter le milieu résultant, et

5) à mesurer le signal émis en cinétique ou à l'équilibre.

5. Procédé selon l'une quelconque des revendications 1 à 4, caractérisé en ce que l'analyte est une substance biologique ou non.

6. Procédé selon l'une quelconque des revendications 1 à 4, caractérisé en ce que l'analyte est choisi parmi les substances biologiques telles que des anticorps, les antigènes, les toxines, les enzymes, les protéines, les hormones, les stéroïdes, l'avidine, la biotine, les micro-organismes et les haptènes et les substances non biologiques susceptibles de se lier de façon spécifique avec un coordinat, telles que les médicaments.

7. Procédé selon l'une des revendications 1 à 5, caractérisé en ce que l'analyte est la prolactine ou l'antigène carcinoembryonnaire.

8. Procédé selon l'une quelconques des revendications 1 à 6, caractérisé en ce que le composé luminescent est un composé fluorescent, chimioluminescent ou phosphorescent.

9. Procédé selon la revendication 7, caractérisé en ce que le composé luminescent est un composé fluorescent choisi parmi la fluorescéine, les cryptates et le chélates de terres rares.

10. Procédé selon l'une quelconque des revendications 1 à 8, caractérisé en ce que l'un des réactifs est marqué à la fluorescéine et l'autre est iodé.

11. Procédé selon l'une quelconque des revendications 1 à 9, caractérisé en ce que le composé luminescent a une longue durée de décroissance luminescente et en ce que l'excitation du milieu résultant est une excitation pulsée.

12. Procédé selon la revendication 10, caractérisé en ce que le composé luminescent est un cryptate de terre rare.

13. Procédé selon l'une quelconque des revendications 1, 3 à 12 pour le dosage d'antigènes ou d'haptènes selon la méthode par compétition, caractérisé en ce qu'il consiste à faire incuber le milieu contenant l'antigène recherché avec l'anticorps correspondant marqué à la fluorescéine en présence d'une quantité donnée d'antigène iodé.

14. Procédé selon l'une quelconque des revendications 1, 3 à 13, pour le dosage d'antigènes ou d'haptènes selon la méthode par compétition, caractérisé en ce qu'il consiste à faire incuber le milieu contenant l'antigène recherché avec l'anticorps correspondant iodé en présence d'une quantité donnée d'antigène marqué à la fluorescéine.

15. Procédé selon l'une quelconque des revendications 1, 2, 5 à 14 pour le dosage d'antigènes ou d'haptènes selon la méthode par excès, caractérisé en ce qu'il consiste à faire incuber le milieu contenant l'antigène recherché avec un premier anticorps marqué à la fluorescéine en présence d'une quantité donnée d'un second anticorps iodé ou vice versa, lesdits anticorps ayant des spécificités différentes pour l'antigène recherché.

16. Coffret pour la détection et/ou la détermination homogène en phase liquide d'un analyte dans un milieu susceptible de le contenir caractérisé en ce qu'il contient :

– un premier réactif constitué d'au moins un récepteur de l'analyte à déterminer ;

– un second réactif constitué d'au moins un des éléments du produit de réaction de l'analyte avec au moins l'un de ses récepteurs, l'un des réactifs étant couplé avec un composé luminescent et l'autre réactif étant couplé avec un atome lourd ou des motifs contenant au moins un atome lourd ;

– des échantillons standard contenant des quantités connues de l'analyte à déterminer pour l'établissement de courbes standard,

– les diluants ou tampons nécessaires au dosage.

17. Coffret selon la revendication 16, caractérisé en ce que le composé luminescent est un composé chimioluminescent et en ce que ledit coffret comprend en outre les réactifs chimiques appropriés nécessaires à l'excitation.

18. Coffret selon l'une des revendications 16 ou 17, pour le dosage de la prolactine ou de l'antigène carcino-embryonnaire.

## Ansprüche

1. Homogenes Detektions-und/oder-Feststellungsverfahren durch Lumineszenz eines Zergliederers in

dafür anfälliger Umgebung, durch Herausstellung des Reaktionsproduktes zwischen Analyten und einem entsprechenden Aufnahmestoff, bezeichnend dadurch, das er darin besteht:

1) der besagten Umgebung einen ersten Reaktivstoff, aus einem Aufnahmestoff des besagten Zergliederers bestehend, zuzufügen ;

2) einen zweiten Reaktivstoff, der aus mindestens einem der Bestandteile des Reaktionsproduktes des Zergliederers mit mindestens einem seiner Aufnahmestoffe besteht, hinzuzufügen, wobei einer der beiden Reaktivstoffe mit einem Schweratom oder einem ein Schweratom enthaltenden gruppen gekoppelt ist ;

3) die daraus entstehende Umgebung entweder jeweils nach Zugabe eines der Reaktivstoffe, oder nach Zugabe beider Reaktivstoffe, brüten zu lassen ;

4) die somit entstandene Umgebung anzureizen, und

5) im Gleichgewicht oder in Kinetik das vom luminiszenten Kompound abgegebene Signal zu messen, wobei dieses Signal durch die Einwirkung des Schweratoms moduliert wird.

2. Verfahren gemäss Anspruch 1, aus einer Überschussmethode bestehend, bezeichnend dadurch, dass es darin besteht:

1) der besagten Umgebung, die den gesuchten Zergliederer enthält, einen ersten, aus einem Aufnehmer des besagten Zergliederers bestehenden, und mit einem luminiszenten Kompound gekoppelten, Reaktivstoff beizufügen ;

2) einen zweiten, aus einem oder mehreren Aufnehmern besagten Zergliederers bestehenden, Reaktivstoff zuzufügen, wobei dieser zweite Reaktivstoff mit einem Schweratom oder einem Schweratom enthaltenden gruppen gekoppelt ist ;

3) die besagte Umgebung nach jeder Reaktivstoffzugabe oder Zugabe eines der Reaktivstoffe brüten zu lassen,

4) die so entstehende Umgebung anzureizen, und

5) das abgegebene Signal in Gleichgewicht oder Kinetik zu messen.

3. Verfahren gemäss Anspruch 1, aus einer Vergleichsmethode bestehend, bezeichnend dadurch, dass es darin besteht:

1) der besagten Umgebung, die den gesuchten Zergliederer enthält, einen aus einem aus dem Aufnehmer des besagten Zergliederers bestehenden Reaktivstoff zuzufügen, mit dem ein Schweratom oder ein Schweratom enthaltende gruppen gekoppelt sind ;

2) einen zweiten, aus dem Zergliederer gekoppelt mit einem Luminiszenzkompound, bestehenden Reaktivstoff zuzufügen ;

3) besagte Umgebung nach jeder Zugabe von Reaktivstoff oder nach Zugabe beider Reaktivstoffe brüten zu Lassen ;

4) die daraus entstehende Umgebung anzureizen, und

5) das davon abgegebene Signal in gleichgewicht oder Kinetik zu messen.

4. Verfahren gemäss Anspruch 1, aus einer Vergleichsmethode bestehend, bezeichnend dadurch, dass es darin besteht:

1) der den gesuchten Zergliederer enthaltenden Umgebung einen Reaktivstoff, bestehend aus einem Aufnehmer besagten Zergliederers, zuzufügen, welcher mit einem Luminiszenzkompound gekoppelt ist ;

2) hierzu, als zweiten Reaktivstoff, den Zergliederer zu fügen, an den ein Schweratom oder ein Schweratom enthaltende gruppen gekoppelt sind ;

3) besagte Umgebung entweder nach Zugabe eines jeden Reaktivstoffes, oder nach Zugabe beider Reaktivstoffe, brüten zu Lassen ;

4) die daraus entstandene Umgebung anzureizen, und

5) das davon abgegebene Signal in Kinetik oder Gleichgewicht zu messen.

5. Verfahren gemäss irgendeinem der Ansprüche 1 bis 4, bezeichnend dadurch, dass der Zergliederer eine biologische oder nichtbiologische Substanz ist.

6. Verfahren gemäss irgendeinem der Ansprüche 1 bis 4, bezeichnend dadurch, dass der Zergliederer unter biologischen Substanzen gewählt wurde, wie Schutzstoffe, Antigene, Giftstoffe, Enzyme, Proteine, Hormone, Steroide, Avidine, Biotine, Mikroorganismen und Haptene, sowie unter nichtbiologischen Substanzen die geeignet sind, sich auf spezifische Weise mit einem Koordinaten, wie Arzneimittel, zu verbinden.

7. Verfahren gemäss einem der Ansprüche 1 bis 5, bezeichnend dadurch dass der Zergliederer Prolactin oder karzinembryonäres Antigen ist.

8. Verfahren gemäss irgendeinem der Ansprüche 1 bis 6, bezeichnend dadurch, dass der Luminiszenzkompound ein fluoreszenter, chemisch leuchtender oder phosphoreszierender Kompound ist.

9. Verfahren gemäss Anspruch 7, bezeichnend dadurch, dass der Lumineszenzkompound ein aus Fluoreszein, Cryptaten und Chelaten von Edelerden gewählt ist.

10. Verfahren gemäss einem der Ansprüche 1 bis 8, bezeichnend dadurch, dass einer der Reaktivstoffe

mit Fluoreszein und der andere mit Jod markiert ist.

11. Verfahren gemäss irgendeinem der Verfahren 1 bis 9, bezeichnend dadurch, dass der Lumineszenzkompound eine lange Dauer der Lumineszenzabschwächung hat, und der Anreiz in daraus entstehender Umgebung ein pulsierender Anreiz ist.

12. Verfahren gemäss Anspruch 10, bezeichnend dadurch, dass der Lumineszenzkompound ein Cryptat aus Edelerde ist.

13. Verfahren gemäss irgendeinem der Ansprüche 1, 3 bis 12 hinsichtlich der Antigen-oder Haptendosierung gemäss der Vergleichsmethode, bezeichnend dadurch, dass es darin besteht, die das die gesuchten Antigene mit entsprechendem Schutzstoff, mit Fluoreszein markiert, in Anwesenheit einer gegebenen Menge jodhaltiger Antigene brüten lässt.

14. Verfahren gemäss einem der Ansprüche 1, 3 bis 13, hinsichtlich der Antigen-oder Haptendosierung gemäss Vergleichsmethode, bezeichnend dadurch, dass es darin besteht, die das gesuchte Antigen mit entsprechendem jodhaltigem Schutzstoff in Anwesenheit einer gegebenen Menge mit Fluoreszein markierten Antigens brüten lässt.

15. Verfahren gemäss irgendeinem der Ansprüche 1, 2, 5 bis 14 hinsichtlich der Dosierung von Antigen oder Hapten gemäss Überschussmethode, bezeichnend dadurch, dass es darin besteht, das gesuchte Antigen mit einem ersten, fluoreszeimarkierten, Schutzstoff in Anwesenheit einer gegebenen Menge eines zweiten, jodhaltigen Schutzstoffes oder umgekehrt, brüten lässt, wobei die besagten Schutzstoffe für das gesuchte Antigen verschiedenartige Eigenarten besitzen.

16. Koffer für homogene Detektion und/oder Feststellung in Flüssigphase eines Zergliederers in dafür anfälliger Umgebung, bezeichnend dadurch, dass er folgendes enthält :

– einen ersten Reaktivstoff bestehend aus mindestens einem Aufnehmer des festzustellenden Zergliederers,

– einen zweiten Reaktivstoff bestehend aus mindestens einem der Bestandteile des Reaktivproduktes des Zergliederers, mit mindestens einem seiner Aufnehmer, wobei einer der Reaktivstoffe mit lumineszentem Kompound, und der andere reaktivstoff mit einem Schweratom oder einem Schweratom enthaltenden gruppen gekoppelt ist,

– Standardmuster enthaltend bekannte Mengen des Zergliederers, festzustellen zur erstellung von Standardkurven,

– die zur Dosierung notwendigen Lösungsmittel oder Tampons.

17. Koffer gemäss Anspruch 16, bezeichnend dadurch, dass der lumineszente Kompound ein chemisch leuchtender Kompound ist, und dass der besagte Koffer ausserdem die zum Anreizen notwendigen, geeigneten chemischen Reaktivstoffe enthält.

18. Koffer gemäss einem der Ansprüche 16 oder 17, zur Dosierung des Prolactins oder karzinembyonären Antigens.

## Claims

1. Homogeneous process for the detection and/or determination of an analyte in a medium in which it may be present, by revealing the reaction product of the analyte and a corresponding receptor, characterized in that it consist in :

1) adding to said medium a first reagent consisting of a receptor for the said analyte ;

2) adding a second reagent consisting of at least one of the components of the reaction product of the analyte and at least one of its receptor ; one of the two reagents being coupled with a luminescent compound and the other reagent being coupled with a heavy atom or units containing a heavy atom ;

3) incubating the medium after addition of each reagent or after the addition of both reagents ;

4) exciting the resulting medium and

5) measuring at equilibrium or during the kinetics, the signal emitted by the luminescent compound, said signal being modulated by the heavy atom effect.

2. Process according to claim 1, which consists of an excess method, characterized in that it consists in

1) adding to the medium containing the target analyte a first reagent consisting of a receptor for the said analyte, coupled with a luminescent compound ;

2) adding a second reagent consisting of one or more additional receptors for the said analyte, the said second reagent being coupled with a heavy atom or units containing a heavy atom ;

3) incubating the medium after addition of each reagent or after the addition of both reagents ;

4) exciting the resulting medium and

5) measuring the signal emitted at equilibrium or during the kinetics.

3. Process according to claim 1, which consists of a competition method, characterized in that it consists in :

1) adding to the medium containing the target analyte a first reagent consisting of a receptor for the said analyte, on which is coupled a heavy atom or units containing a heavy atom ;
2) adding a second reagent consisting of the analyte coupled with a luminescent compound ;
3) incubating the medium after addition of each reagent or after the addition of both reagents ;
4) exciting the resulting medium and
5) measuring the signal emitted at equilibrium or during the kinetics.

4. Process according to claim 1, which consists of a competition method, characterized in that it consists in :

1) adding to the medium containing the target analyte a first reagent consisting of a receptor for the said analyte, the said receptor being coupled with a luminescent compound ;
2) adding, as a second reagent, the analyte on which is coupled a heavy atom or units containing a heavy atom ;
3) incubating the medium after addition of each reagent or after the addition of both reagents ;
4) exciting the resulting medium and
5) measuring the signal emitted during the kinetics or at equilibrium.

5. Process according to any one of claims 1 to 4, characterized in that the analyte is a biological substance, or a non-biological substance.

6. Process according to any one of claims 1 to 4, characterized in that the analyte is selected among the group consisting of antibodies, antigens, toxins, enzymes, proteins, hormones, steroids, avidin, biotin, microorganisms and haptens and non-biological substances capable of binding specifically with a ligand, such as drugs.

7. Process according to any one of claims 1 to 5, characterized in that the analyte is prolactin or carcinoembryonic antigen.

8. Process according to any one of claims 1 to 6, characterized in that the luminescent compound is a fluorescent, chemoluminescent or phosphoroscent compound.

9. Process according to claim 7, characterized in that the luminescent compound is a fluorescent compound chosen from the group consisting of fluorescein and rare earth cryptates and chelates.

10. Process according to any one of claims 1 to 8, characterized in that one of the reagents is labelled with fluorescein and the other is iodinated.

11. Process according to any one of claims 1 to 9, characterized in that the luminescent compound has a long luminescent decay lifetime and in that excitation of the resulting medium is a pulsed excitation.

12. Process according to claim 10, characterized in that the luminescent compound is a rare earth cryptate.

13. Process according to any one of claims 1 and 3 to 12, for the determination of antigens or haptens by the competition method, characterized in that it consists in incubating the medium containing the target antigen with the corresponding fluorescein-labelled antibody in the presence of a given quantity of iodinated antigen.

14. Process according to any one of claims 1 and 3 to 13, for the determination of antigens or haptens by the competition method, characterized in that it consists in incubating the medium containing the target antigen with the corresponding iodinated antibody in the presence of a given quantity of fluorescein-labelled antigen.

15. Process according to any one of claims 1, 2 and 5 to 14, for the determination of antigens or haptens by the excess method, characterized in that it consists in incubating the medium containing the target antigen with a first fluorescein-labelled antibody in the presence of a given quantity of a second iodinated antibody, or vice versa, the said antibodies having different specificities for the target antigen.

16. A kit for the homogeneous detection and/or determination in liquid phase of an analyte in a medium in which it may be present, characterized in that it contains :

- a first reagent consisting of at least one receptor for the analyte to be determined ;
- a second reagent consisting of at least one of the components of the reaction product of the analyte and at least one of its receptors, one of the reagents being coupled with a luminescent compound and the other reagent being coupled with a heavy atom or units containing at least one heavy atom ;
- standard samples containing known quantities of the analyte to be determined, for establishing standard curves ; and
- the diluents or buffers required for the determination.

17. Kit according to claim 16, characterized in that the luminescent compound is a chemoluminescent compound and wherein the said kit also comprises the appropriate chemical reagents required for excitation.

18. Kit according to one of claims 16 or 17, for the determination of the prolactin or the carcinoembryonic antigen.

Fig-1

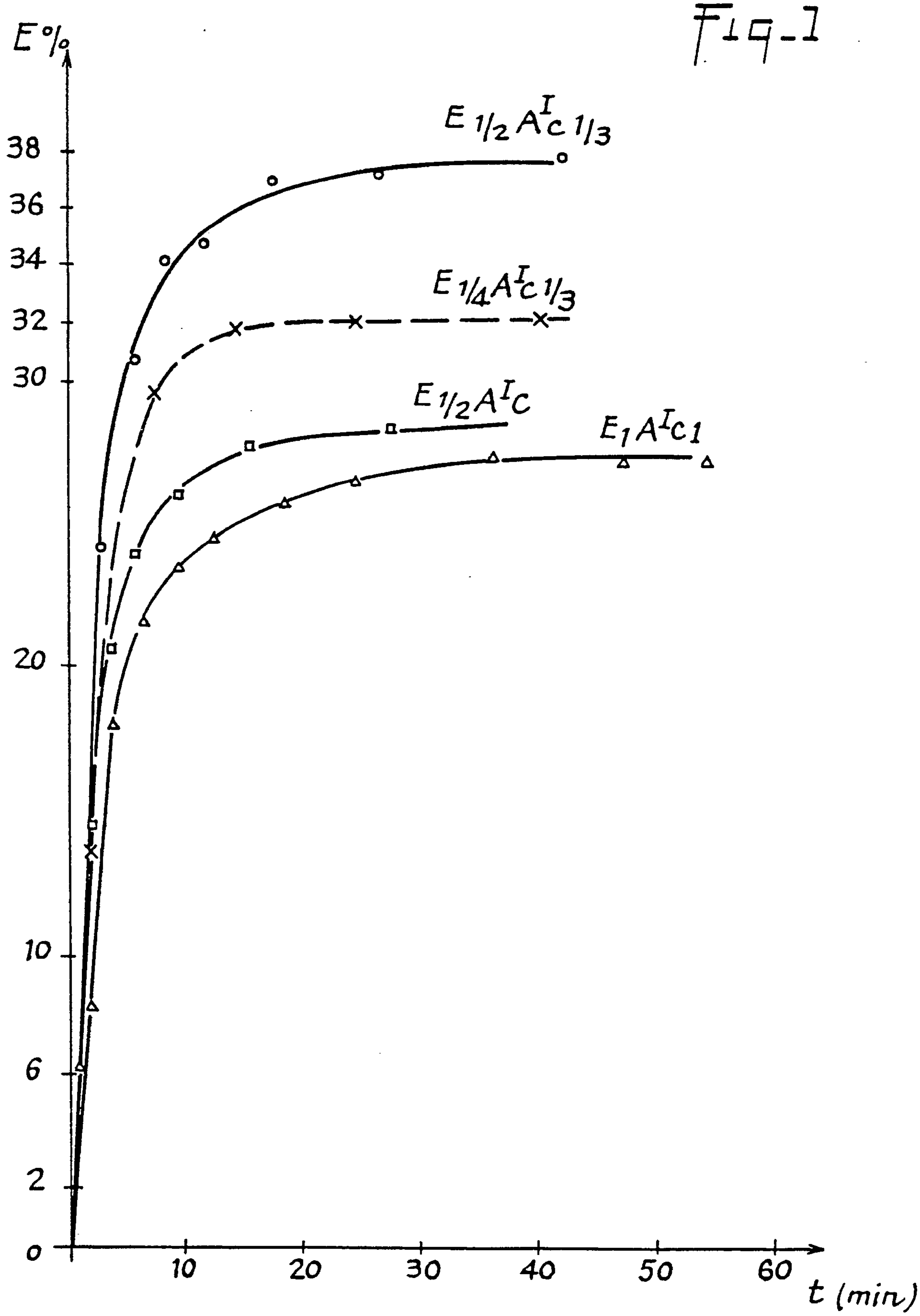
E%
38
36
34
32
30
20
10
6
2
0
10
20
30
40
50
60
t (min)
E 1/2 A^I c 1/3
E 1/4 A^I c 1/3
E 1/2 A^I c
E 1 A^I c 1

Fig-2